# EUROPÄISCHE PATENTSCHRIFT

(11) **EP 4 171 438 B1**
(45) Veröffentlichungstag und Bekanntmachung des Hinweises auf die Patenterteilung: **20.08.2025**
(21) Anmeldenummer: 21805455.9
(22) Anmeldetag: 02.11.2021
(51) Int. Cl.: A61C 17/06, A61M 1/00

(54) **ABSCHEIDER MIT INTEGRIERTEM DICHTELEMENT ZUR FLÜSSIGKEIT-LUFT-TRENNUNG**
SEPARATOR COMPRISING AN INTEGRATED SEALING ELEMENT FOR FLUID-AIR SEPARATION
SÉPARATEUR COMPRENANT UN ÉLÉMENT D'ÉTANCHÉITÉ INTÉGRÉ POUR LA SÉPARATION FLUIDE-AIR

(30) Priorität: 02.11.2020 AT 509412020
(43) Veröffentlichungstag der Anmeldung: 03.05.2023
(73) Patentinhaber: Pregenzer, Bruno, 6414 Mieming (AT)
(72) Erfinder: Pregenzer, Bruno, 6414 Mieming (AT)
(74) Vertreter: Laufhütte, Dieter
(86) Internationale Anmeldenummer: PCT/EP2021/080292
(87) Internationale Veröffentlichungsnummer: WO 2022/090552

(56) Entgegenhaltungen:
- EP-B1- 0 108 983
- DE-A1- 10 139 026
- DE-U1- 29 906 470
- SE-C2- 502 307
- US-A- 5 018 971

## Beschreibung

Die vorliegende Erfindung betrifft einen Abscheider zur Flüssigkeit-Luft-Trennung eines Abwassergemisches, insbesondere einen Dentalabscheider zur Flüssigkeit-Luft-Trennung eines zahnärztlichen Abwassergemisches, nach dem Oberbegriff des Anspruchs 1.

Solche Abscheider kommen beispielsweise im zahnärztlichen Bereich zum Einsatz und dienen der Trennung unterschiedlicher Phasen eines aus einer zahnärztlichen Absaugeinrichtung oder Speischale stammenden Abwassergemisches. Das Abwassergemisch kann beispielsweise dem Abscheider als Drei-Phasen-Gemisch mit flüssigen, gasförmigen und festen Bestandteilen zugeführt und durch diesen in die unterschiedlichen Bestandteile aufgetrennt werden. Ebenfalls sind Abscheider bekannt, die lediglich eine Flüssigkeit-Luft-Trennung des Abwassergemisches durchführen, wobei entweder das Abwassergemisch von vorneherein keine festen Bestandteile enthält oder diese ohne Trennung zusammen mit der Flüssigkeit abgeführt werden.

Ein Dentalabscheider zur Drei-Phasen-Trennung ist beispielsweise aus der WO 92/18062 bekannt. Hierbei erfolgt die Trennung der Feststoffpartikel aus der Flüssigkeit mittels einer Vollmantelzentrifuge, während die aus der Zentrifuge übertretende Flüssigkeit über einen Flüssigkeitsauslass abfließt. Nach jeder Zentrifugierphase flie-ßen die Feststoffpartikel mit einem Restflüssigkeitsanteil über einen Feststoffablass in einen unterhalb der Zentrifuge angeordneten, abnehmbaren Sedimentationsbehälter. Zur Abtrennung der Luft ist der Dentalabscheider mit einem zahnärztlichen Luftabscheider verbunden, wobei die Abdichtung des Unterdruckbereichs über ein Rückschlagventil erfolgt, welches entweder vor Eintritt des Abwassergemisches in den Dentalabscheider oder nach Austritt der abgetrennten Flüssigkeit aus dem Abscheider in der entsprechenden Einlass- oder Auslassleitung angeordnet ist.

EP 108 983 B1, SE 502 307 C2, US 5 018 971 A, DE 101 39 026 A1 und DE 299 06 470 U1 werden als relevanter Stand der Technik zitiert.

Die vorliegende Erfindung setzt sich nun zur Aufgabe, eine konstruktiv einfache und kompakte Lösung für die Flüssigkeit-Luft-Trennung bei derartigen Abscheidern zur Verfügung zu stellen.

Erfindungsgemäß wird diese Aufgabe durch eine Vorrichtung mit den Merkmalen des Anspruchs 1 gelöst. Vorteilhafte Ausführungsformen der Erfindung ergeben sich aus den Unteransprüchen und der nachfolgenden Beschreibung.

Demnach wird ein Abscheider zur Flüssigkeit-Luft-Trennung eines insbesondere zahnärztlichen Abwassergemischs, also insbesondere ein Dentalabscheider, vorgeschlagen. Der Abscheider weist ein Gehäuse mit einem Gemischeinlass für das zu trennende Abwassergemisch, einem Flüssigkeitsauslass für aus dem Abwassergemisch abgetrennte Flüssigkeit und einem Luftablass zum Absaugen von Luft aus dem Inneren des Gehäuses auf. Bei dem Abwassergemisch kann es sich um ein Zwei-Phasen- oder ein Drei-Phasen-Gemisch handeln. Ferner ist der hierin verwendete Begriff "Luft" breit auszulegen und bezeichnet generell ein Gas mit beliebiger Zusammensetzung, welches von den übrigen Komponenten des Gemisches abgetrennt bzw. abgesaugt werden soll.

Erfindungsgemäß ist innerhalb des Gehäuses ein Dichtelement vorgesehen, welches den Flüssigkeitsauslass luftdicht von einem mit dem Luftablass und dem Gemischeinlass in Verbindung stehenden Innenvolumen des Gehäuses trennt. Das Dichtelement weist mindestens ein Ventilelement auf, welches dazu ausgelegt ist, einen Durchtritt von Flüssigkeit aus dem Innenvolumen nach außen zuzulassen und ein Eindringen von Luft durch das Dichtelement in das Innenvolumen zu verhindern.

Bei der vorgenannten durch das Dichtelement tretenden Flüssigkeit kann es sich um eine Flüssigkeit des Abwassergemisches mit darin enthaltenen festen Bestandteilen bzw. Feststoffpartikeln (beispielsweise vor einem weiteren Abtrennen mittels einer Zentrifuge), um eine Flüssigkeit des Abwassergemisches, welche bereits beim Eintritt in den Gemischeinlass keine festen Bestandteile enthielt, oder um eine von festen Bestandteilen abgetrennte Flüssigkeit des Abwassergemisches handeln.

Die Integration eines Dichtelements zur Flüssigkeit-Luft-Trennung in das Gehäuse des Abscheiders ermöglicht einen kompakten und einfachen Aufbau. Hierbei wird die Grenze des Unterdruckbereichs (d.h. desjenigen Volumens, innerhalb welchem ein Unterdruck zum Absaugen der Luft herstellbar ist - im vorliegenden Fall ist dies das sogenannte Innenvolumen) in das Gehäuse verlegt. Hierdurch werden z.B. Ausgestaltungen ermöglicht, bei der ein Teil der Abtrennung der Abwassergemischbestandteile innerhalb und ein anderer Teil außerhalb des Unterdruckbereichs erfolgt.

Der Flüssigkeitsauslass kann unterhalb des Innenvolumens, beispielsweise in einem unteren Bereich bzw. an einer Unterseite des Gehäuses, angeordnet sein. Eine solche Ausgestaltung kommt beispielsweise in Betracht, wenn mittels des erfindungsgemäßen Abscheiders lediglich eine Flüssigkeit-Luft-Trennung durchgeführt werden soll. Alternativ kann der Flüssigkeitsauslass mit einer umlaufenden Auslasskammer verbunden sein, welche beispielsweise ringförmig um das Innenvolumen umläuft bzw. rundumlaufend ausgebildet ist. Diese Konfiguration kann z.B. bei einer Flüssigkeit-Feststoff-Luft-Trennung sinnvoll sein, wenn insbesondere der Abscheider zusätzlich eine Zentrifuge mit einer drehbaren Zentrifugentrommel umfasst. In all diesen möglichen Konfigurationen kann mittels des erfindungsgemäßen Dichtelements eine einfache und effektive Flüssigkeit-Luft-Separation erfolgen. Der Begriff "ringförmig umlaufen" ist breit auszulegen und kann z.B. bedeuten, dass die Auslasskammer in Draufsicht kreisförmig umläuft.

In einer Ausführungsform ist vorgesehen, dass das mindestens eine Ventilelement ausgelegt ist, ein Eindringen von Luft von außen in das Innenvolumen in Kombination mit einem im Innenvolumen herrschenden Unterdruck zu verhindern, wobei der Unterdruck insbesondere mittels einer an den Luftablass angeschlossenen oder anschließbaren Luftabsaugvorrichtung bzw. einem Luftabscheider erzeugbar ist. Der Unterdruck führt insbesondere zu einem selbstständigen Schließen des mindestens einen Ventilelements, wobei ein Durchtreten von Flüssigkeit nach außen unter Beibehaltung der Luftdichtheit immer noch möglich ist.

In einer weiteren Ausführungsform ist eine Verteilervorrichtung vorgesehen, mittels welcher Flüssigkeit gegen das mindestens eine Ventilelement drückbar oder schleuderbar ist, damit diese das Dichtelement durchdringen kann. Die Verteilervorrichtung erzeugt also insbesondere einen für ein Passieren des Ventilelements notwendigen Flüssigkeitsdruck, welcher entgegen einem im Innenvolumen herrschenden Unterdruck eine Öffnung des Ventilelements bewirkt. Die Verteilervorrichtung umfasst vorzugsweise ein rotatorisch antreibbares Element, bei dem es sich beispielsweise um einen Gemischverteiler im Mündungsbereich des Gemischeinlasses oder um eine Zentrifugentrommel bzw. damit verbundene Trennschaufeln handeln kann.

In einer weiteren Ausführungsform ist vorgesehen, dass das Dichtelement zumindest abschnittsweise aus einem elastischen Material, insbesondere Gummi, gefertigt ist, wobei das mindestens eine Ventilelement durch eine Ventilklappe gebildet ist, die durch durchtretende Flüssigkeit insbesondere reversibel nach außen auslenkbar und vorzugsweise einstückig im elastischen Material ausgebildet ist. Vorzugsweise ist eine Vielzahl (d.h. mindestens zwei) von auf Umfang gleichmäßig verteilten Ventilklappen vorgesehen.

Im einfachsten Fall ist die Ventilklappe bzw. sind die Ventilklappen durch Einschnitte in das elastische Material gebildet. Sie werden durch die gegen das Dichtelement geschleuderte bzw. gedrückte Flüssigkeit nach außen gebogen und lassen Flüssigkeit durchtreten. Aufgrund der Rückstellkraft des elastischen Materials werden die Ventilklappen ohne den von innen ausgeübten Druck der Flüssigkeit selbstständig wieder geschlossen und gedichtet, insbesondere gegen einen im Inneren bzw. im Innenvolumen herrschenden Unterdruck, sodass eine luftdichte Abdichtung besteht. Es kann vorgesehen sein, dass das gesamte Dichtelement aus dem elastischen Material besteht und beispielsweise als Gummiring bzw. -band ausgebildet ist, oder dass nur einzelne Bereiche, z.B. die Ventilklappen, aus dem elastischen Material bestehen. Die Ventilklappen weisen vorzugsweise eine rechteckige Form auf.

Durch diese Gestaltung des Dichtelements ergibt sich eine konstruktiv einfache und kostengünstig herzustellende Lösung für die dynamische Luft-Flüssigkeit-Trennung.

In einer weiteren Ausführungsform ist vorgesehen, dass das Dichtelement an einem innerhalb des Innenvolumens angeordneten Stützelement anliegt, in welchem mindestens eine jeweils einer Ventilklappe zugeordnete Durchtrittsöffnung ausgebildet ist. Bei dem Stützelement kann es sich beispielsweise um einen im Bereich der Übertrittsöffnung zwischen dem Dichtelement und der Zentrifugenkammer angeordneten Trennsteg handeln, wie weiter unten genauer beschrieben ist. Alternativ kann es sich dabei um ein Ventilgehäuse (oder ein mit diesem verbundenes Bauteil) handeln, welches einen im Bereich des Gemischeinlasses und/oder Luftablasses angeordneten Gemischverteiler umgibt, wie ebenfalls weiter unten genauer beschrieben ist. Das Stützelement kann gleichzeitig der Fixierung des Dichtelements dienen. Ferner kann das Stützelement Teil einer das Innenvolumen begrenzenden Wand / Abgrenzung sein, wobei das Stützelement auch in einem solchen Fall als im obigen Sinne "innerhalb des Innenvolumens angeordnet" gilt.

Vorzugsweise ist die mindestens eine Durchtrittsöffnung kleiner als die zugeordnete Ventilklappe, damit letztere nicht nach innen öffnen und dadurch Luft von außen einströmen lassen kann. Dadurch werden die Ventilklappen bei einem im Innenvolumen herrschenden Unterdruck und Abwesenheit von nach außen drückender Flüssigkeit von außen gegen das Stützelement gedrückt und dichten das Innenvolumen luftdicht nach außen ab.

In einer weiteren Ausführungsform ist ein rotatorisch antreibbarer Gemischverteiler vorgesehen, welcher innerhalb des Innenvolumens drehbar gelagert ist, wobei das aus dem Gemischeinlass strömende Abwassergemisch mittels des Gemischverteilers verteilbar und/oder nach außen schleuderbar ist und wobei der Gemischverteiler vorzugsweise von der Seite gesehen eine im Wesentlichen konische Form aufweist, die sich insbesondere nach unten verbreitert. Der Gemischverteiler kann aus einem Kunststoff bestehen.

In einer weiteren Ausführungsform ist vorgesehen, dass der Gemischverteiler als Laufrad mit einer Mehrzahl (d.h. mindestens zwei) von insbesondere geschwungenen Trennschaufeln bzw. Trennflügeln ausgebildet ist. In Draufsicht weist das Laufrad vorzugsweise eine runde Form auf. Vorzugsweise ist das Abwassergemisch über einen ersten Bereich des Laufrads verteilbar und die aus dem Innenvolumen abgesaugte Luft über einen zweiten Bereich des Laufrads zum Luftablass leitbar, wobei der erste Bereich bevorzugt vom zweiten Bereich räumlich getrennt ist (wobei allerdings eine Überlappung der beiden Bereiche denkbar ist). So kann vorgesehen sein, dass das Abwassergemisch mit einem innen / außen liegenden Bereich des Laufrads in Berührung kommt, während die Luft über einen außen / innen liegenden Bereich geleitet wird.

In einer weiteren Ausführungsform ist vorgesehen, dass der Gemischverteiler mindestens einen durchgängigen Luftkanal umfasst und derart im Bereich der Mündung des Luftablasses angeordnet ist, dass die aus dem Innenvolumen angesaugte Luft durch den mindestens einen Luftkanal in den Luftablass strömt, wobei der mindestens eine Luftkanal vorzugsweise im Wesentlichen parallel zur Drehachse des Gemischverteilers verläuft. Es können eine Vielzahl von Luftkanälen vorgesehen sein, die beispielsweise zumindest teilweise zwischen einer Mehrzahl von Trennschaufeln verlaufen. Die Trennschaufeln können also einerseits dem Verteilen des Abwassergemisches und andererseits dem Leiten von angesaugter Luft dienen. Alternativ oder zusätzlich können eigens für den Luftabzug vorgesehene Luftkanäle ausgebildet sein. Der Gemischverteiler kann unmittelbar am / im Luftablass angeordnet sein.

In einer weiteren Ausführungsform ist vorgesehen, dass der Gemischverteiler zumindest teilweise von einem rotationsstarr mit dem Gehäuse verbundenen Verteilergehäuse umgeben ist, welches mit dem Luftablass und/oder dem Gemischeinlass verbunden ist und mindestens eine Durchtrittsöffnung für das Abwassergemisch aufweist. Das Verteilergehäuse schließt vorzugsweise unmittelbar an den Luftablass und/oder den Gemischeinlass an, wobei dessen Innen- und/oder Außenkontur bevorzugt eine konische Form aufweist, die insbesondere der Außenkontur des Gemischverteilers folgt. Der Luftabzug kann zentral und der Gemischeinlass um den Luftablass herum angeordnet sein oder umgekehrt. Dadurch wird beispielsweise das Abwassergemisch an einen äußeren Bereich des Gemischverteilers geleitet, an dem sich insbesondere mehrere Trennschaufeln befinden, während die Luft aus dem Innenvolumen über einen inneren / mittigen Bereich des Gemischverteilers angesaugt wird. Das Verteilergehäuse kann nach unten offen sein, kann aber auch anderweitig ausgebildet sein, beispielsweise geschlossen mit seitlichen Durchtrittsöffnungen.

In einer weiteren Ausführungsform ist vorgesehen, dass das Gehäuse eine Zentrifugenkammer aufweist, in welcher eine rotatorisch antreibbare Zentrifugentrommel drehbar gelagert ist. Mittels der Zentrifuge können im Abwassergemisch enthaltene Feststoffpartikel von der Flüssigkeit getrennt werden. Der Gemischeinlass und der Luftabzug münden hierbei in die Zentrifugenkammer, wobei zwischen der Oberseite der Zentrifugentrommel und dem Gehäuse eine Übertrittsöffnung derart ausgebildet ist, dass während des Zentrifugiervorgangs Flüssigkeit von der Zentrifugentrommel in eine mit dem Flüssigkeitsauslass in Verbindung stehende Auslasskammer gelangen kann. Vorzugsweise ist der Gemischeinlass und/oder der Luftabzug zentral oberhalb der Zentrifugentrommel angeordnet.

Bei der Zentrifugentrommel handelt es sich vorzugsweise um einen als Vollmantelzentrifuge ausgebildeten Zentrifugenbehälter, welcher insbesondere über eine Motoreinheit in Drehung versetzt wird, sodass die im Abwassergemisch enthaltenen Feststoffe wie z.B. Amalgampartikel aufgrund der Zentrifugalkraft an die Innenwand gedrückt werden, während die Flüssigkeit über die Übertrittsöffnung in die Auslasskammer gelangen kann. Dadurch wird eine Feststoff-Flüssigkeit-Trennung erreicht. Bei der Zentrifugenkammer handelt es sich insbesondere um einen innerhalb des Gehäuses ausgebildeten und vorzugsweise im Wesentlichen zylindrisch geformten Hohlraum, in welchem die Zentrifugentrommel drehbar gelagert ist.

Die oberhalb der Zentrifugentrommel ausgebildete Übertrittsöffnung kann als Teil der Zentrifugenkammer angesehen werden, erstreckt sich aber insbesondere radial über die Zentrifugenkammer nach außen und bildet eine Verbindung mit der Auslasskammer. Letztere kann eine im Wesentlichen ringförmige bzw. rundumlaufende Kammer darstellen, von der ein beispielsweise Anschluss für den Flüssigkeitsablass abgeht. Die Auslasskammer kann einen geneigten bzw. abgeschrägten Boden aufweisen, sodass die sich darin sammelnde Flüssigkeit selbstständig in den Flüssigkeitsauslass fließt. Letzterer kann tangential von der Auslasskammer abgehen. Die Auslasskammer ist insbesondere rotationsstarr, ebenso wie die Zentrifugenkammer und die Übertrittsöffnung.

Die Zentrifugentrommel kann konisch geformt sein, d.h. sich nach oben zur Übertrittsöffnung hin verbreitern, um einen Austritt der Flüssigkeit zu erleichtern. Alternativ oder zusätzlich kann die Zentrifugentrommel eine insbesondere im oberen Bereich der Zentrifugentrommel angeordnete Rückhaltevorrichtung bzw. einen Flüssigkeitsfang aufweisen, welcher beispielsweise einen umlaufenden und nach unten weisenden Steg umfassen kann. Dadurch wird verhindert, dass Feststoffe bei einer Drehung oben aus der Zentrifugentrommel treten können. Erst wenn die sich an der Innenwand der Zentrifugentrommel bildende Flüssigkeitsschicht eine gewisse Dicke bzw. Stärke erreicht, tritt Flüssigkeit über den Flüssigkeitsfang in die Übertrittsöffnung, während die Feststoffe in der Zentrifugentrommel verbleiben.

Die Auslasskammer, die Zentrifugenkammer, die Zentrifugentrommel und/oder der Gemischverteiler können koaxial zueinander angeordnet sein. Für die Auslasskammer bedeutet dies beispielsweise, dass sie ringförmig um die Zentrifugenkammer umlaufen kann, wobei die geometrische Mitte der kreisförmigen Kammer auf der Drehachse der Zentrifugentrommel liegt. Dadurch ergibt sich ein kompakter Aufbau des erfindungsgemäßen Abscheiders.

In einer weiteren Ausführungsform ist vorgesehen, dass der oben beschriebene Gemischverteiler innerhalb der Zentrifugentrommel drehbar gelagert und vorzugsweise zusammen mit dieser über eine Welle rotatorisch antreibbar ist. Dabei ist der Gemischverteiler ausgebildet, das aus dem Gemischeinlass tretende Abwassergemisch gegen die Innenwandung der Zentrifugentrommel oder - falls das Dichtelement im Bereich des Gemischverteilers angeordnet ist - gegen das Dichtelement zu schleudern.

In einer weiteren Ausführungsform ist vorgesehen, dass an der Zentrifugentrommel im Bereich der Übertrittsöffnung eine Mehrzahl von vorzugsweise auf Umfang verteilten Trennschaufeln angeordnet sind, welche dazu ausgebildet sind, die während des Zentrifugiervorgangs oben aus der Zentrifugentrommel austretende Flüssigkeit nach außen zu schleudern. Die Trennschaufeln sind insbesondere geschwungen. Ferner sind die Trennschaufeln vorzugsweise an einem Flansch ausgebildet oder angebracht, welcher mit einem oberen Rand der Zentrifugentrommel fest oder lösbar verbunden ist. Der sich mit der Zentrifugentrommel mitdrehende Flansch ragt vorzugsweise am oberen Ende der Zentrifugentrommel radial nach außen und ist insbesondere zumindest teilweise in einem zwischen Auslasskammer und Zentrifugenkammer ausgebildeten Spalt angeordnet, welcher Teil der Übertrittsöffnung ist.

Darüber hinaus ist es vorstellbar, dass der Flansch einen in Umfangrichtung umlaufenden Steg aufweist, welcher in einer zwischen Auslasskammer und Zentrifugenkammer ausgebildeten Nut verläuft, wobei vorzugsweise der Steg und die Nut eine Labyrinthdichtung bilden oder Teil einer solchen sind. Der Steg und die Nut sind insbesondere von oben gesehen kreisförmig und laufen um die vorzugsweise ebenfalls in Draufsicht kreisförmige Zentrifugenkammer um. Der Steg dreht sich mit der Zentrifugentrommel mit und gleitet in der Nut. Ein Teil der Außenwand der Zentrifugentrommel kann ebenfalls Teil der Labyrinthdichtung sein, ebenso wie ggf. weitere Dichtelemente oder Stege / Nuten. Durch die Labyrinthdichtung wird eine optimale Abdichtung am Rand des Flüssigkeitsablaufs bzw. Flüssigkeitsaustritts erreicht.

Für den Fall, dass die Trennschaufeln im Unterdruckbereich, d.h. im Innenvolumen angeordnet sind, was bedeutet, dass das Dichtelement außerhalb der Zentrifugentrommel angeordnet ist (und diese beispielsweise rundumlaufend umgibt), fungieren die Trennschaufeln als Hilfspumpe, welche entgegen dem Unterdruck die Flüssigkeit gegen die Ventilklappe(n) des Dichtelements schleudert und den zu deren Öffnung notwendigen Druck erzeugt. Für den Fall, dass das Dichtelement innerhalb der Zentrifugentrommel angeordnet ist und sich die Trennschaufeln somit außerhalb des Innenvolumens befinden, können diese dem Verteilen bzw. Fördern der abgetrennten Flüssigkeit in Richtung Auslasskammer dienen.

In einer weiteren Ausführungsform ist vorgesehen, dass das Dichtelement im Bereich der Übertrittsöffnung angeordnet ist und vorzugsweise die Zentrifugentrommel ringförmig umgibt, wobei die Zentrifugenkammer und zumindest ein Abschnitt der Übertrittsöffnung Teil des Innenvolumens, d.h. Teil des Unterdruckbereichs sind. Hier erfolgt die Trennung der festen Bestandteile des Abwassergemisches innerhalb des Innenvolumens. Bei dieser Ausführungsform kann das oben beschriebene Stützelement, an welchem das Dichtelement anliegt, als Trennsteg ausgeführt sein, welcher zwischen dem Dichtelement und der Zentrifugenkammer angeordnet ist. Der Trennsteg kann einstückig mit dem Gehäuse, beispielsweise einem öffenbaren Gehäusedeckel, ausgebildet sein oder ein separates Bauteil darstellen, welches im / am Gehäuse gelagert bzw. montiert ist. Der Trennsteg kann gleichzeitig der Fixierung des Dichtelements dienen, beispielsweise indem er am Gehäuse / Gehäusedeckel angeordnet ist und beim Schließen des Deckels einen Teil des Dichtelements, beispielsweise eine umlaufende Befestigungslippe, einklemmt.

In einer alternativen Ausführungsform ist vorgesehen, dass das Dichtelement im Bereich der Durchtrittsöffnung des Verteilergehäuses angeordnet ist und vorzugsweise das Verteilergehäuse ringförmig bzw. rundumlaufend umgibt. Bei dieser Ausführungsform bildet also ein Teil des Verteilergehäuses selbst (oder ein mit diesem verbundenes separates Bauteil) das oben beschriebene Stützelement, an dem das Dichtelement von außen anliegt. Falls eine Zentrifuge zur Trennung eines Drei-Phasen-Gemisches vorgesehen ist, erfolgt bei dieser Ausführungsform die Trennung bzw. Abscheidung der festen Bestandteile des Abwassergemisches außerhalb des Unterdruckbereichs. Allerdings ist hier auch eine einfache Zwei-Phasen-Trennung bzw. Flüssigkeit-Luft-Separation denkbar, also eine Ausführungsform ohne Zentrifuge, wobei die aus dem Dichtelement austretende Flüssigkeit z.B. direkt in einen Flüssigkeitsauslass abfließen kann.

In einer weiteren Ausführungsform ist vorgesehen, dass die Zentrifugentrommel einen Feststoffauslass aufweist, mit welchem ein mit dem Gehäuse abnehmbar bzw. abmontierbar verbundener Absetz- bzw. Sedimentationsbehälter verbunden ist. Der Sedimentationsbehälter dient der Sammlung von aus dem Abwassergemisch abgetrennten und nach einem Zentrifugiervorgang unter Schwerkrafteinwirkung absinkenden Feststoffpartikeln. Vorzugsweise umfasst der Abscheider ferner eine insbesondere gemeinsam mit der Zentrifugentrommel und/oder dem Gemischverteiler antreibbare Pumpe bzw. Pumpenvorrichtung, mittels welcher eine im Sedimentationsbehälter enthaltene Flüssigkeit zur Abtrennung fester Bestandteile in die Zentrifugentrommel förderbar ist. Bei besagter Flüssigkeit kann es sich um ein Abwassergemisch mit darin enthaltenen Feststoffbestandteilen handeln.

Hier ist einerseits eine Konfiguration möglich, bei der das Abwassergemisch zuerst durch einen Direkteinlass in den Sedimentationsbehälter geleitet wird, in dem vor allem größere Teile der festen Bestandteile absinken. Die überstehende Flüssigkeit, in der kleinere, d.h. feine und feinste feste Bestandteile enthalten sind, die beispielsweise aufgrund einer zu kurzen Verweilzeit noch nicht sedimentieren konnten oder wieder aufgewirbelt wurden, wird in die Zentrifugentrommel überführt. Hierfür kann die mittels der Pumpe nach oben zur Zentrifuge geförderte Flüssigkeit dem Gemischeinlass zugeführt werden, welche im Abscheider als "Abwassergemisch" entsprechend der zuvor beschriebenen Ausführungsbeispiele verarbeitet wird. Für das über den direkten Einlass in den Sedimentationsbehälter geleitete Abwassergemisch erfolgt insbesondere also keine Abtrennung der Luft.

Andererseits kann dem zuvor beschriebenen Direkteinlass ein anderes Abwassergemisch zugeführt werden (beispielsweise aus einer zahnärztlichen Speischale o.Ä.) als dem oben im Bereich des Luftablasses angeordneten Gemischeinlass (diesem kann beispielsweise ein Abwassergemisch einer zahnärztlichen Absaugvorrichtung bzw. eines Saugschlauchs zugeführt werden).

Die Pumpe kann ein koaxial unterhalb der Zentrifugentrommel und/oder unterhalb des Gemischverteilers angeordnetes, drehbar gelagertes und rotatorisch antreibbares Ansaugteil umfassen. Dieses kann mittels einer eigenen Motoreinheit oder durch die zum Antrieb der Zentrifugentrommel verwendete Motoreinheit antreibbar sein. Das Ansaugteil kann als Flügelrad mit beispielsweise von der Seite gesehen konischer Form ausgebildet sein. Ebenfalls ist es vorstellbar, dass das Ansaugteil ein insbesondere konisch nach unten verjüngter und in den Sedimentationsbehälter hineinragender Teil der Zylindertrommel ist, beispielsweise ein Ansaugkonus. In letzterem Fall erfolgt automatisch beim Zentrifugieren immer auch ein Ansaugen von überstehender Flüssigkeit (falls diese eine gewisse Mindestfüllhöhe erreicht hat) in die Zentrifugentrommel, sodass die enthaltenen festen Bestandteile von der Flüssigkeit abgetrennt werden. Am Ende des Zentrifugiervorgangs fallen bzw. gelangen die abgetrennten festen Bestandteile wieder über den zum Sedimentationsbehälter hin offenen Fortsatz bzw. Ansaugkonus, welcher gleichzeitig den Feststoffauslass bildet oder umfasst, in den Sedimentationsbehälter.

Weitere Merkmale, Einzelheiten und Vorteile der Erfindung ergeben sich aus den nachfolgend anhand der Figuren erläuterten Ausführungsbeispielen. Es zeigen:
- Figur 1:: eine seitliche Schnittansicht des erfindungsgemäßen Abscheiders gemäß einem ersten Ausführungsbeispiel;
- Figur 2:: den Abscheider gemäß Figur 1 in einer perspektivischen Ansicht;
- Figur 3:: eine seitliche Schnittansicht des erfindungsgemäßen Abscheiders gemäß einem zweiten Ausführungsbeispiel;
- Figur 4:: den Abscheider gemäß Figur 3 in einer perspektivischen Ansicht;
- Figur 5:: Teile des Abscheiders gemäß Figur 3 in einer Explosionsansicht;
- Figur 6:: eine seitliche Schnittansicht des erfindungsgemäßen Abscheiders gemäß einem dritten Ausführungsbeispiel;
- Figur 7:: den Abscheider gemäß Figur 6 in einer perspektivischen Ansicht; und
- Figur 8:: Teile des Abscheiders gemäß Figur 6 in einer Explosionsansicht.

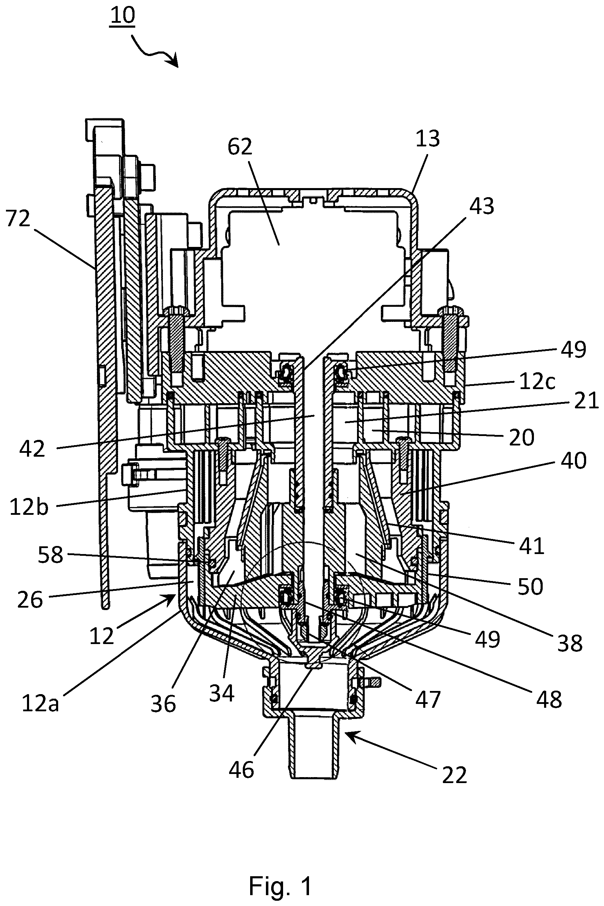

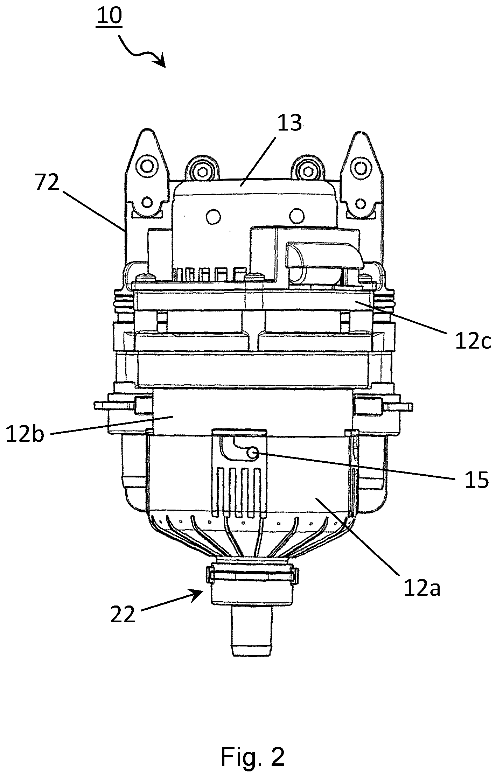

Die Figur 1 zeigt einen zentralen Schnitt durch ein erstes Ausführungsbeispiel des erfindungsgemäßen Abscheiders 10 in einer seitlichen Ansicht. Eine perspektivische Ansicht des Abscheiders 10 ist in der Figur 2 gegeben. Die hierin verwendeten Begriffe "oben" und "unten" beziehen sich auf den Fall, dass der Abscheider 10 auf einer ebenen Fläche abgestellt ist oder sich in einer ebenen Einbaulage befindet und daher so ausgerichtet ist, wie es in der Figur 1 gezeigt ist.

Der erfindungsgemäße Abscheider 10 gemäß dem ersten Ausführungsbeispiel dient der Flüssigkeit-Luft-Trennung eines Abwassergemisches und weist keine Zentrifuge auf. Der Abscheider 10 besitzt ein Gehäuse 12, welches in ein Unterteil 12a mit einem an der Unterseite zentral angeordneten Flüssigkeitsauslass 22, ein Mittelteil 12b und ein mit dem Mittelteil 12b verschraubtes Oberteil 12c unterteilt ist. Ober- und Mittelteil 12b, 12c sind miteinander verschraubt, während das Unterteil 12a über L-förmige Aussparungen mit seitlich vom Mittelteil 12b abstehenden Pins 15 verbunden und dadurch auf das Mittelteil 12b aufgesteckt werden kann (vgl. Figur 2). Oberhalb des Oberteils 12c befindet sich ein von einer Motorabdeckung 13 umgebener Motor 62, welcher der Übersichtlichkeit halber bis auf die Umrisse ausgeblendet ist. Der Motor 62 treibt eine Welle 42 rotatorisch an, welche sich von oben nach unten zentral durch den gesamten Abscheider 10 bis in den Bereich des Flüssigkeitsauslasses 22 erstreckt. Die Welle 42 ist bis auf die Umrisse ebenfalls ausgeblendet.

Zwischen dem Oberteil 12c und dem Mittelteil 12b ist ein Gemischeinlass 20 ausgebildet, welcher im Wesentlichen die Form eines um die Welle 42 umlaufenden Ringraums aufweist und mit einem außen am Gehäuse 12 angeordneten Anschluss für einen Schlauch o.Ä. verbunden ist. Der Gemischeinlass 20 mündet in einen durch Unter- und Mittelteil 12a, 12b gebildeten Innenraum und dient der Zuführung des zu trennenden Abwassergemisches, welches beispielsweise über einen mit dem Anschluss verbundenen Schlauch einer zahnärztlichen Absaugvorrichtung oder einer Speischale bereitgestellt wird. Der durch Unter- und Mittelteil 12a, 12b gebildete Innenraum ist im Wesentlichen zylindrisch geformt und verjüngt sich im unteren (durch das Unterteil 12a gebildeten) Bereich konisch zum Flüssigkeitsauslass 22 hin.

Ein Luftabzug 21, welcher ebenfalls im Wesentlichen die Form eines um die Welle 42 umlaufenden Ringraums aufweist, ist innerhalb des Gemischeinlasses 20 angeordnet und mit einem weiteren außen am Gehäuse 12 angeordneten Anschluss für einen Schlauch o.Ä. verbunden. Gemischeinlass 20, Luftabzug 21 und Welle 42 sind koaxial zueinander angeordnet. Mittels einer hier nicht dargestellten Luftansaugvorrichtung bzw. eines Luftabscheiders kann über den Luftablass 21 Luft aus dem Inneren des Gehäuses 12 abgesaugt und so vom Abwassergemisch getrennt werden.

In dem durch Unter- und Mittelteil 12a, 12b gebildeten Innenraum ist ein Gemischverteiler 36 drehbar gelagert, welcher als Laufrad mit einer Vielzahl von gleichmäßig auf Umfang verteilten und insbesondere gebogenen Trennschaufeln ausgebildet ist und von der Seite gesehen eine sich nach unten verbreiternde und im Wesentlichen konische Form aufweist. Das Laufrad 36 ist mit der Welle 42 drehfest verbunden und dadurch vom Motor 62 antreibbar. Zwischen dem Laufrad 36 und dem Oberteil 12c verläuft die Welle 42 in einem hohlzylindrischen Distanzstück 43, welches sich mit der Welle 42 mitdreht und über eine umlaufende Lagerung 49 im Oberteil 12c drehbar gelagert (und insbesondere abgedichtet) ist.

Das Laufrad 36 ist zentral unterhalb des Gemischeinlasses 20 und des Luftabzugs 21 angeordnet und von einem nicht-drehbar mit dem Mittelteil 12b verbundenen Verteilergehäuse 40 umgeben, wobei die Innenkontur des Verteilergehäuses 40 der konischen Außenkontur des Laufrads 36 folgt. Das Verteilergehäuse 40 weist ferner eine konische Innenwandung 41 auf und ist mit dem Gemischeinlass 20 und dem Luftabzug 21 derart verbunden, dass die Innenwandung 41 den mit dem Luftabzug 21 verbundenen inneren Bereich des Verteilergehäuses 40 von dem mit dem Gemischeinlass 20 verbundenen äußeren Bereich trennt. Die Innenwandung 41 reicht dabei nicht bis an das untere Ende des Verteilergehäuses 40 und bildet mit dessen Außenwandung einen umlaufenden und mit dem Gemischeinlass 20 verbundenen Spalt.

Das einfließende Abwassergemisch strömt von oben aus dem Gemischeinlass 20 in den Spalt zwischen Innenwandung 41 und Außenwandung des Verteilergehäuses 40 und trifft anschließend auf die Trennschaufeln im äußeren Bereichs des Laufrads 36, welche sich insbesondere unterhalb der Innenwandung 41 von innen nach außen erstrecken. Das Abwassergemisch fließt dabei in zwischen den Trennschaufeln au-ßen ausgebildete Zwischenräume und wird, da sich das Laufrad 36 schnell dreht, nach außen geschleudert.

Das nach unten offene Verteilergehäuse 40 ist von unten durch ein Stützelement 34 abgedeckt, welches das Verteilergehäuse 40 nach unten luft- und flüssigkeitsdicht verschließt und im Verbindungsbereich durch ein Dichtungselement bzw. O-Ring 58 abgedichtet ist. Das Laufrad 36 ist vollständig von Verteilergehäuse 40 und Stützelement 34 umgeben und dreht sich innerhalb des dadurch gebildeten Innenraums. Das Stützelement 34 weist seitlich mehrere (hier nicht sichtbare) Durchtrittsöffnungen auf, durch die das mittels des Laufrads 36 nach außen geschleuderte Abwassergemisch dringen kann.

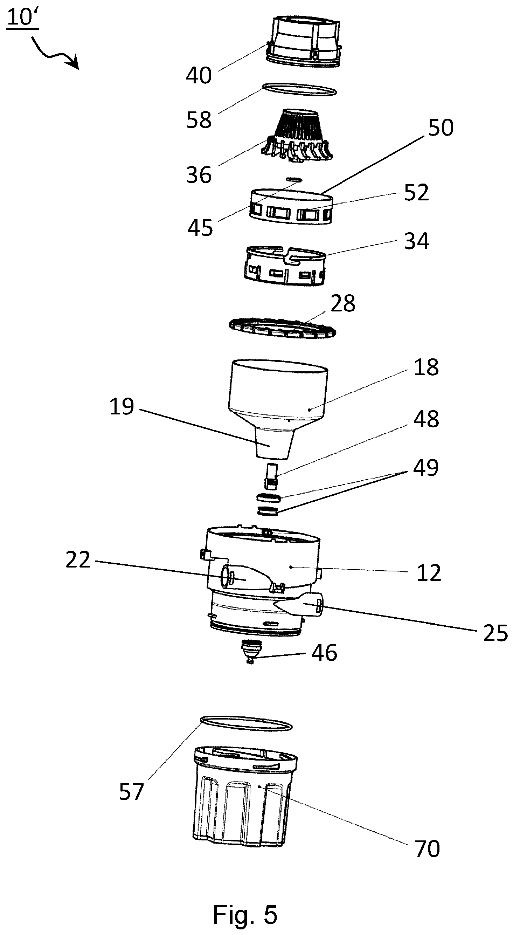

Außen am Stützelement 34 liegt ein Dichtelement 50 an, welches das Stützelement 34 ring- bzw. bandförmig umgibt. Das Verteilergehäuse 40, der O-Ring 58, das Laufrad 36, das Stützelement 34 und das Dichtelement 50 sind in der Figur 5 in einer Explosionsdarstellung perspektivisch dargestellt. Das Dichtelement 50 besteht aus einem flexiblen Material wie Gummi und weist eine Vielzahl von gleichmäßig auf Umfang verteilten Ventilelementen 52 auf, die als biegbare Ventilklappen 52 ausgebildet sind. Jeder Ventilklappe 52 ist dabei eine Durchtrittsöffnung des Stützelements 34 zugeordnet und dieser benachbart angeordnet, sodass diese sich überdecken. Die rechteckigen Ventilklappen 52 sind durch (eckige) U-förmige Einschnitte in das flexible Material gebildet und lassen sich durch den Druck des mittels des Laufrads 36 nach außen geschleuderten Abwassergemisches reversibel nach außen biegen.

Der Luftablass 21 ist mit dem durch die Innenwandung 41 abgetrennten inneren Bereich des Verteilergehäuses 40 verbunden, sodass die Luft über innerhalb der Innenwandung 41 verlaufende Luftkanäle 38 des Laufrads 36 abgesaugt wird. Diese Luftkanäle 38 sind durch Zwischenräume von sich radial erstreckenden und insbesondere seitlich gebogenen Trennschaufeln gebildet. Die Endbereiche zumindest einiger dieser Trennschaufeln dienen der Verteilung des Abwassergemisches im äußeren Bereich des Verteilergehäuses 40. Durch die Drehung des Laufrads 36 und die Trennung des inneren Bereichs des Luftabzugs vom äußeren Bereich des Abwassergemischflusses mittels der Innenwandung 41 wird verhindert, dass Flüssigkeit in den Luftablass 21 gesaugt wird.

Das Dichtelement 50 dichtet das durch Verteilergehäuse 40 und Stützelement 34 gebildete Innenvolumen luftdicht ab. Dadurch, dass über den Luftablass 21 die Luft aus dem Innenvolumen abgesaugt wird, herrscht darin ein Unterdruck. Durch diesen Unterdruck werden die Ventilklappen 52 von außen gegen das Stützelement 34 gepresst, sodass die darin ausgebildeten Durchlassöffnungen bzw. Fenster luftdicht abgeschlossen sind. Die Flüssigkeit des Abwassergemisches (welche Feststoffpartikel enthalten oder aber eine rein flüssige Phase darstellen kann) wird durch die Trennschaufeln des Laufrads 36 von innen gegen die Ventilklappen 52 geschleudert. Die Trennschaufeln fungieren somit als eine Art Hilfspumpe, damit die Flüssigkeit den Unterdruck überwinden und in den Bereich außerhalb des Innenvolumens bzw. Unterdruckbereichs gelangen kann.

Aufgrund ihrer Elastizität biegen sich durch den Druck der Flüssigkeit die Ventilklappen 52 nach außen und ermöglichen einen Durchtritt der Flüssigkeit. Dabei kann jedoch keine Luft nach außen entweichen oder von außen in das Innenvolumen gelangen, da aufgrund der Rückstellkraft des flexiblen Materials die Ventilklappen 52 sofort wieder gegen den Unterdruck geschlossen werden, sobald keine Flüssigkeit mehr von innen dagegen drückt. Beim Durchtritt der Flüssigkeit dichtet diese selbst die geöffneten Ventilklappen 52 luftdicht ab. Das Dichtelement 50 ermöglicht somit eine einfache, effektive und kostengünstige Flüssigkeit-Luft-Trennung innerhalb des Gehäuses 12.

Nachdem die Flüssigkeit das Dichtelement 50 passiert hat, fließt sie über den konisch geformten Bereich des Gehäuses 12 nach unten in den Flüssigkeitsauslass 22. Zum Schutz der Welle 42, die über ein mittels einer Mutter 47 fixiertes Kupplungsteil 48 mit dem Laufrad 36 verbunden ist, sind das Ende der Welle 42 und die Mutter 47 durch eine Abdeckkappe 46 abgedeckt. Das Kupplungsteil 48 ist über ein rundumlaufendes Lager 49 in dem rotationsstarren Stützelement 34 drehbar gelagert und gegenüber diesem abgedichtet. Der Abscheider 10 kann über eine Halterung 72 an der Wand oder einer anderen Vorrichtung befestigt werden, wobei die Halterung 72 einen Schwingungsdämpfer umfassen kann.

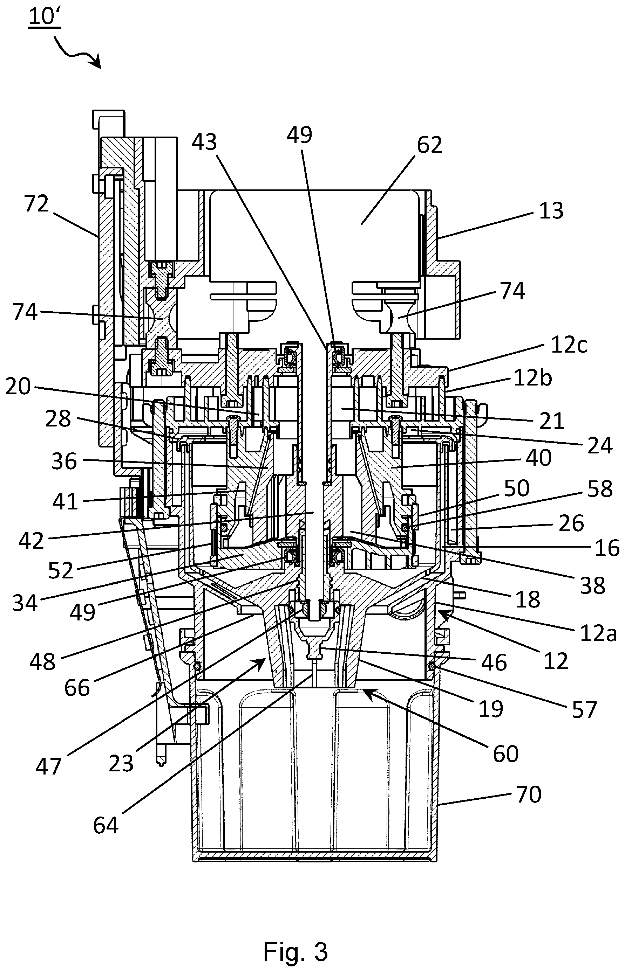

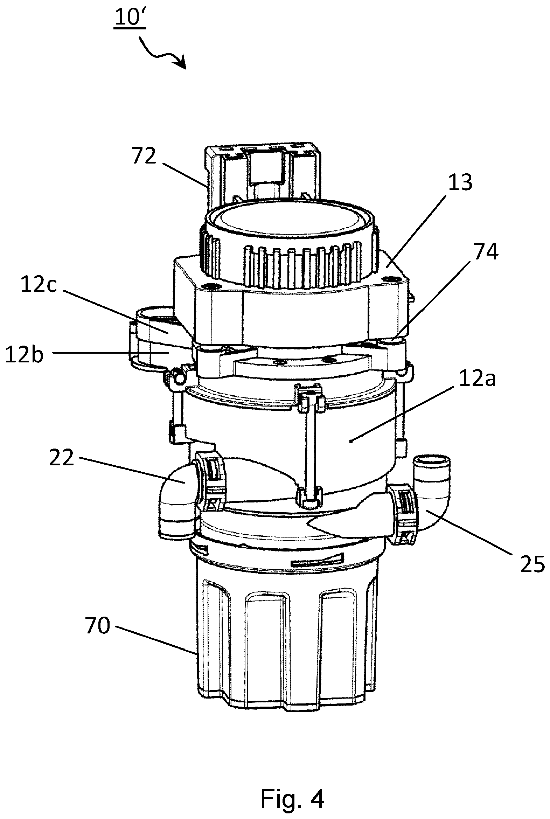

Ein zweites Ausführungsbeispiel des erfindungsgemäßen Abscheiders 10' ist in den Figuren 3-4 dargestellt, wobei die Figur 3 einen zentralen Schnitt durch den Abscheider 10' in einer seitlichen Ansicht, die Figur 4 eine perspektivische Ansicht des Abscheiders 10' und die Figur 5 eine Explosionsansicht einiger der Komponenten des Abscheiders 10' zeigen. Bauteile mit bereits im Rahmen des ersten Ausführungsbeispiels verwendeten Bezugszeichen erfüllen bei diesem Abscheider 10' dieselbe Funktion, sodass auf eine wiederholende detaillierte Beschreibung verzichtet wird.

Das Gehäuse 12 weist ein nach unten offenes Unterteil 12a auf, in welchem analog zum ersten Ausführungsbeispiel das Laufrad 36, das umgebende Verteilergehäuse 40, das Stützelement 34 und das Dichtelement 50 angeordnet sind. Oberhalb des Unterteils 12a sind in einem Mittelteil 12b der Gemischeinlass 20 und der Luftabzug 21 ausgebildet, wobei das Mittelteil 12b von einem Oberteil 12c abgedeckt ist (die zwischen den äußeren Anschlüssen und dem Gemischeinlass 20 bzw. Luftabzug 21 verlaufenden Kanäle können im Mittelteil 12b, im Oberteil 12c oder in Mittel- und Oberteil 12b, 12c ausgebildet sein). Die Flüssigkeit-Luft-Trennung mittels des erfindungsgemäßen Dichtelements 50 funktioniert analog zum ersten Ausführungsbeispiel. Der oberhalb des Oberteils 12c befestigte Motor 62 ist mittels mehrere Dämpfungselemente 74 schwingungsgedämpft.

Im Unterschied zum ersten Ausführungsbeispiel weist dieser Abscheider 10' eine Zentrifuge zum Abtrennen fester Bestandteile aus dem Abwassergemisch auf, sodass eine 3-Phasen-Trennung erfolgt. Hierfür bildet das Unterteil 12a eine Zentrifugenkammer 16, in welcher eine als nach oben offene Vollmantelzentrifuge ausgebildete Zentrifugentrommel 18 (auch als Zentrifugenbehälter bezeichnet) drehbar gelagert ist. Die Zentrifugentrommel 18 wird zusammen mit dem Laufrad 36 durch den Motor 62 über die Welle 42 angetrieben. Dabei ist die Zentrifugentrommel 18 über ein entsprechend geformtes Kupplungsteil 48 mit der Welle 42 drehfest verbunden. Zwischen Kupplungsteil 48 und Laufrad 36 ist eine Beilagscheibe 45 angeordnet (vgl. Figur 5).

Die Zentrifugentrommel 18 umgibt das Verteilergehäuse 40 mit dem Dichtelement 50, sodass das durch das Dichtelement 50 tretende Flüssigkeitsgemisch in die Zentrifugentrommel 18 gelangt und aufgrund der Zentrifugalkraft der rotierenden Zentrifugentrommel 18 gegen deren Innenwand gedrückt wird. Dabei verbleiben die festen Bestandteile bzw. Feststoffpartikel an der Innenwand, während die Flüssigkeit oben aus der Zentrifugentrommel 18 austritt und über eine Übertrittsöffnung 24 in eine um die Zentrifugenkammer 16 umlaufende Auslasskammer 26 gelangt. Die Auslasskammer 26 ist mit einem tangentialen Flüssigkeitsauslass 22 verbunden (vgl. Figur 4), wobei die sich in der Auslasskammer 26 sammelnde Flüssigkeit aufgrund einer Abschrägung ihres Bodens selbstständig in den Flüssigkeitsauslass 22 fließt. An diesen kann ein geeigneter Schlauch angeschlossen werden.

Die Zentrifugentrommel 18 kann leicht konisch geformt sein, um den Übertritt von Flüssigkeit aus der Zentrifugentrommel 18 über die Übertrittsöffnung 24 in die Auslasskammer 26 zu erleichtern. Wie insbesondere in der Figur 5 zu erkennen ist, weist die Zentrifugentrommel 18 am oberen Rand einen sich mit dieser mitdrehenden Flansch 28 auf, der vorzugsweise an der Oberseite mit einer Vielzahl von Trennschaufeln versehen ist, die die aus der Zentrifugentrommel 18 austretende Flüssigkeit nach außen in die Auslasskammer 26 fördern. Ferner ragt der Flansch 28 radial ein Stück weit nach innen und bildet dadurch einen Flüssigkeitsfang. Dieser sorgt dafür, dass die gegen die Innenwand der Zentrifugentrommel 18 gedrückten Feststoffpartikel nicht (mit einer gewissen Menge Restflüssigkeit) oben aus der Zentrifugentrommel 18 treten. Erst wenn sich ein ausreichend starker Flüssigkeitsfilm an der Innenwand der Zentrifugentrommel 18 gebildet hat, welcher über die Breite des Flüssigkeitsfangs hinausgeht, tritt Flüssigkeit oben aus der Zentrifugentrommel 18 aus. Die ganz außen befindlichen Feststoffpartikel verbleiben dadurch stets in der Zentrifugentrommel 18. Der Flansch 18 bzw. die Zentrifugentrommel 18 kann gegenüber dem Gehäuse 12 über eine Labyrinthdichtung abgedichtet sein.

Nach jedem Zentrifugiervorgang fließen die Feststoffpartikel aufgrund der Schwerkraft (und der nun nicht mehr wirkenden Zentrifugalkraft) mit einem Anteil von Restflüssigkeit nach unten und gelangen über einen am Boden der Zentrifugentrommel 18 vorgesehenen Feststoffauslass 23 in einen mit dem Unterteil 12a verbundenen und darunter angeordneten Sedimentationsbehälter 70. An ihrer Unterseite ist die Zentrifugentrommel 18 konisch nach unten zum Feststoffauslass 23 hin verjüngt. Der abnehmbare Sedimentationsbehälter 70 ist oben offen und schließt direkt an das nach unten offene Unterteil 12a des Gehäuses 12 an. Die Abdichtung erfolgt über ein als O-Ring ausgebildetes Dichtungselement 57. Im Bereich der konischen Verjüngung der Zentrifugentrommel 18 weist das Unterteil 12a einen ebenfalls konisch geformten Durchlass 66 auf, welcher die Zentrifugentrommel 18 bzw. deren konischen Fortsatz 19 umgibt und zum Sedimentationsbehälter 70 hin offen ist.

In den Sedimentationsbehälter 70 kann über einen eigens am Unterteil 12a vorgesehenen Direkteinlass 25 ein Abwassergemisch geleitet werden. Der Direkteinlass 25 mündet knapp oberhalb des Durchlasses 66 in das Unterteil 12a, sodass das aus dem Direkteinlass 25 eintretende Abwassergemisch durch den Durchlass 66 direkt in den Sedimentationsbehälter 70 gelangt. Dem Direkteinlass 25 kann somit ein anderes Abwassergemisch zugeführt werden (beispielsweise aus einer zahnärztlichen Speischale) als dem Gemischeinlass 20 (diesem kann beispielsweise ein Abwassergemisch eines zahnärztlichen Saugschlauchs zugeführt werden), wobei nur dem durch den Gemischeinlass 20 geleiteten Abwassergemisch die Luft entzogen bzw. abgetrennt wird.

Der Feststoffauslass 23 ist in einem sich vom Boden der Zentrifugentrommel 18 nach unten erstreckenden konischen Fortsatz bzw. Konus 19 ausgebildet, der gleichzeitig als Pumpe 60 fungiert, welche überstehende Flüssigkeit aus dem Sedimentationsbehälter 70 nach oben in die Zentrifugentrommel 18 fördert. Dieser hier als Ansaugkonus 19 bezeichnete Fortsatz weist an der Innenseite mehrere in Längsrichtung verlaufende Rippen auf und dreht sich gemeinsam mit der Zentrifugentrommel 18 um die durch die Welle 42 gebildete Drehachse. Der Ansaugkonus 19 ragt ein Stück weit in den Sedimentationsbehälter 70 hinein.

Sobald die Flüssigkeit im Sedimentationsbehälter 70 eine gewisse Füllhöhe auf Höhe des Ansaugkonus 19 erreicht (welche z.B. über eine hier nicht näher dargestellte Sensorvorrichtung erfassbar sein kann), wird sie beim Betrieb der Zentrifuge, also wenn sich die Zentrifugentrommel 18 dreht, durch den mitdrehenden Ansaugkonus 19 automatisch in die Zentrifugentrommel 18 gefördert. Dadurch werden in der überstehenden Flüssigkeit enthaltene Feststoffpartikel in der Zentrifugentrommel 18 abgetrennt, wobei diese nach Beendigung des Zentrifugiervorgangs wieder durch den Ansaugkonus 19 bzw. den Feststoffauslass 23 zurück in den Sedimentationsbehälter 70 absinken. Insbesondere können über den Direkteinass 25 und den Gemischeinlass 20 gleichzeitig zwei verschiedene Abwassergemische aufgetrennt werden, da die Pumpe 60 gleichzeitig mit der Zentrifugentrommel 18 angetrieben wird. Sobald ein maximaler Füllstand von Feststoffpartikeln im Sedimentationsbehälter 70 erreicht ist, kann dieser vom Gehäuse 12 abgenommen, verschlossen und z.B. einer Weiterverarbeitung bzw. Entsorgung der darin gesammelten Feststoffe zugeführt werden.

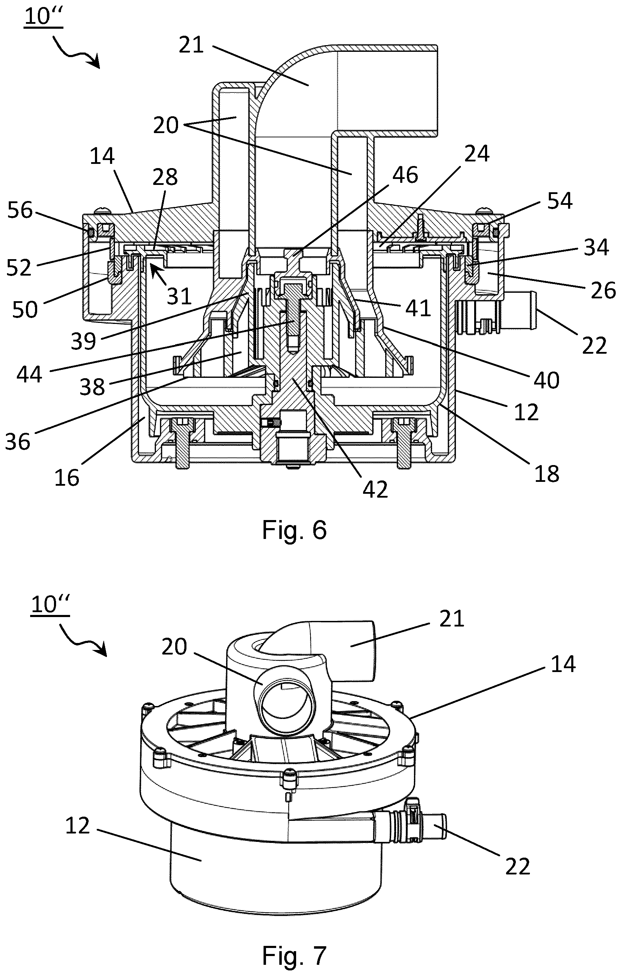

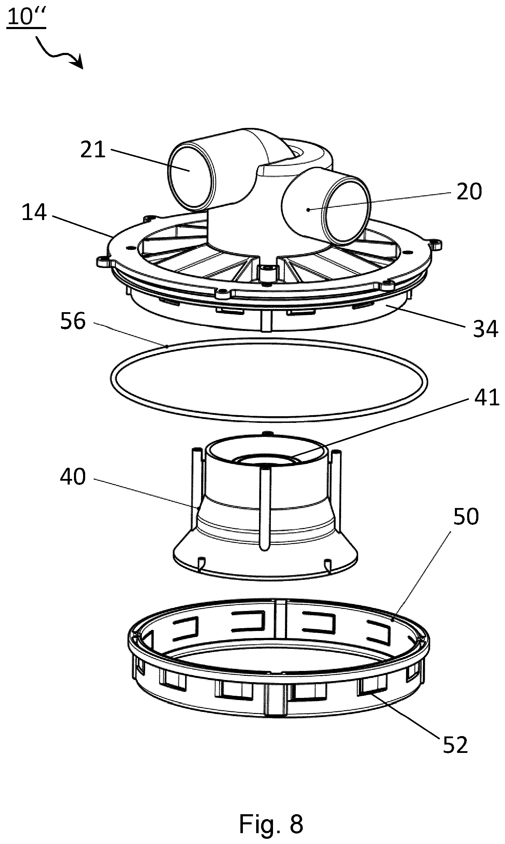

Ein drittes Ausführungsbeispiel des erfindungsgemäßen Abscheiders 10" ist in den Figuren 6-8 dargestellt, wobei die Figur 6 einen zentralen Schnitt durch den Abscheider 10" in einer seitlichen Ansicht, die Figur 7 eine perspektivische Ansicht des Abscheiders 10" und die Figur 8 eine Explosionsansicht einiger der Komponenten des Abscheiders 10" zeigen. Bauteile mit bereits im Rahmen der ersten beiden Ausführungsbeispiele verwendeten Bezugszeichen erfüllen bei diesem Abscheider 10" dieselbe Funktion, sodass auf eine wiederholende detaillierte Beschreibung verzichtet wird.

Auch mittels dieses Abscheiders 10" ist eine Drei-Phasen-Trennung eines über einen zentralen Gemischeinlass 20 zugeführten Abwassergemisches möglich. Im Unterschied zum zweiten Ausführungsbeispiel weist dieser Abscheider 10" allerdings keinen separaten Sedimentationsbehälter auf, sondern die durch die Zentrifugentrommel 18 abgeschiedenen Feststoffe verbleiben in der unten geschlossenen Zentrifugentrommel 18. Der obere Teil des Gehäuses 12 ist als abnehmbarer Deckel 14 ausgebildet, sodass die Zentrifugentrommel 18 ausbaubar und ggf. zusammen mit den darin gesammelten Feststoffbestandteilen verschickbar ist (insbesondere nach Verschließen der Zentrifugentrommel 18 mit einem Trommeldeckel).

Im Gehäuse 12 ist eine im Wesentlichen zylindrische Zentrifugenkammer 16 ausgebildet, in der die Zentrifugentrommel 18 drehbar gelagert ist. Die Zentrifugentrommel 18 ist mit einer im Gehäuse 12 drehbar gelagerten und über eine hier nicht näher dargestellte Motoreinheit rotatorisch antreibbaren Welle 42 drehfest verbunden, wobei die Welle 42 von unten durch das Gehäuse 12 hindurchgeführt ist. Die Abdichtung zwischen Gehäuseunterteil und Deckel 14 erfolgt über ein als O-Ring ausgebildetes Dichtungselement 56.

Der Luftablass 21 befindet sich zentral oberhalb der Zentrifugenkammer 16 und ist vom Gemischeinlass 20 umgeben. Analog zu den anderen Ausführungsbeispielen ist an den Gemischeinlass 20 und den Luftabzug 21 ein Verteilergehäuse 40 angebunden, innerhalb welchem ein drehbares Laufrad 36 drehbar gelagert ist. Im Unterschied zu den ersten beiden Ausführungsbeispielen ist das Verteilergehäuse 40 nach unten offen und weist kein Stützelement 34 bzw. Dichtelement 50 auf. Durch Drehung des Laufrads 36 wird das Abwassergemisch verteilt und nach unten / außen gegen die Innenwand der Zentrifugentrommel 18 geschleudert.

Das Dichtelement 50 ist hier im Bereich einer die Zentrifugenkammer 16 mit der umlaufenden Auslasskammer 24 verbindenden Übertrittsöffnung 24 angeordnet. Das Stützelement 34 ist als umlaufender Trennsteg 34 ausgebildet, welcher am Gehäusedeckel 14 befestigt ist und das Dichtelement 50 über eine nach innen abstehende Befestigungslippe im Gehäuse 12 einklemmt und dadurch fixiert. Der Trennsteg 34 mit seinen Durchtrittsöffnungen ist in der Figur 8 gut zu erkennen.

Während bei den ersten beiden Ausführungsbeispielen das unter Unterdruck stehende und durch das Dichtelement 50 luftdicht abgedichtete Innenvolumen auf den Innenbereich des Verteilergehäuses 40 begrenzt ist, gehören bei diesem Ausführungsbeispiel auch die Zentrifugenkammer 16 bzw. der durch die Zentrifugentrommel 18 eingefasste Bereich sowie ein Teil der Übertrittsöffnung 24 zum Innenvolumen bzw. Unterdruckbereich. Im Gegensatz zum zweiten Ausführungsbeispiel, wo die Abtrennung der festen Bestandteile außerhalb des Unterdruckbereichs stattfindet, wird in diesem Ausführungsbeispiel die Abtrennung der Feststoffpartikel in der Zentrifugentrommel 18 also unter Unterdruck durchgeführt.

Um den für die Durchführung der abgetrennten Flüssigkeit durch das Dichtelement 50 notwendigen Druck zu erzeugen, weist die Zentrifugentrommel 18 am oberen Rand einen Flansch 28 mit einer Vielzahl von auf Umfang gleichmäßig verteilten, seitlich geschwungenen Trennschaufeln auf. Der Flansch 28 ragt radial nach innen und schließt mit einem nach unten weisenden, umlaufenden Steg ab, welcher einen Flüssigkeitsfang 31 bildet und dessen Funktion oben erläutert wurde. Am radial nach außen weisenden Abschnitt des Flansches 28 ist ein in einer Gehäusenut verlaufender Steg angeordnet, welche eine Labyrinthdichtung bilden.

Zwischen der Oberseite des Flansches 28 und dem Gehäusedeckel 14 verbleibt ein geringer Spalt, welcher Teil der Übertrittsöffnung 24 ist, die sich als um die Zentrifugenkammer 16 umlaufender Spalt bis zur Auslasskammer 26 fortsetzt. In diesem Spalt 24 bewegen sich die Trennschaufeln des Flansches 28. Durch die Drehung des Flansches 28 zusammen mit der Zentrifugentrommel 18, wird die oben aus der Zentrifugentrommel 18 austretende Flüssigkeit zwischen die Trennschaufeln gedrängt und durch diese nach außen gegen die Ventilklappen 52 des Dichtelements 50 geschleudert. Die Trennschaufeln des Flansches 28 fungieren somit als Hilfspumpe.

Der Trennsteg 34, das Dichtelement 50 und das Ventilgehäuse 40 sind am Deckel 14 befestigt. Bevor die Zentrifugentrommel 18 ausgebaut werden kann, muss das Laufrad 36 herausgenommen werden, welches mittels einer Schraube 44 an der Welle 42 befestigt ist. Die Schraube 44 ist von der Abdeckkappe 46 abgedeckt und ermöglicht ein einfaches und schnelles Entfernen des Laufrads 36.

Alternativ kann in dem Abscheider 10" gemäß dem dritten Ausführungsbeispiel auch das Dichtelement 50 am Ventilgehäuse 40 angebracht sein, analog zu den ersten beiden Ausführungsbeispielen.

### Bezugszeichenliste:

- 10: Abscheider
- 12: Gehäuse
- 12a: Unterteil
- 12b: Mittelteil
- 12c: Oberteil
- 13: Motorabdeckung
- 14: Deckel
- 15: Pin
- 16: Zentrifugenkammer
- 18: Zentrifugentrommel
- 19: Ansaugkonus
- 20: Gemischeinlass
- 21: Luftablass
- 22: Flüssigkeitsauslass
- 23: Feststoffauslass
- 24: Übertrittsöffnung
- 25: Direkteinlass
- 26: Auslasskammer
- 28: Flansch
- 31: Flüssigkeitsfang
- 34: Stützelement
- 36: Gemischverteiler (Laufrad)
- 38: Luftkanal
- 39: Konischer Bereich
- 40: Verteilergehäuse
- 41: Innenwandung
- 42: Welle
- 43: Distanzstück
- 44: Schraube
- 45: Beilagscheibe
- 46: Abdeckkappe
- 47: Mutter
- 48: Kupplungsteil
- 49: Lagerung
- 50: Dichtelement
- 52: Ventilelement (Ventilklappe)
- 54: Nut
- 56: Dichtungselement
- 57: Dichtungselement
- 58: Dichtungselement
- 60: Pumpe
- 62: Motor
- 64: Rippe
- 66: Durchlass
- 70: Sedimentationsbehälter
- 72: Halterung
- 74: Dämpfungselement

## Patentansprüche

1. Abscheider (10) zur Flüssigkeit-Luft-Trennung eines insbesondere zahnärztlichen Abwassergemischs, umfassend ein Gehäuse (12) mit einem Gemischeinlass (20) für das zu trennende Abwassergemisch, einem Flüssigkeitsauslass (22) für aus dem Abwassergemisch abgetrennte Flüssigkeit und einem Luftablass (21) zum Absaugen von Luft aus dem Inneren des Gehäuses (12),
wobei ein innerhalb des Gehäuses (12) angeordnetes Dichtelement (50), welches den Flüssigkeitsauslass (22) luftdicht von einem mit dem Luftablass (21) und dem Gemischeinlass (20) in Verbindung stehenden Innenvolumen des Gehäuses (12) trennt und
**dadurch gekennzeichnet, dass** das Dichtelement (50) mindestens ein Ventilelement (52) aufweist, welches ausgelegt ist, einen Durchtritt von Flüssigkeit aus dem Innenvolumen nach außen zuzulassen und ein Eindringen von Luft durch das Dichtelement (50) in das Innenvolumen zu verhindern.

2. Abscheider (10) nach Anspruch 1, **dadurch gekennzeichnet, dass** das mindestens eine Ventilelement (52) ausgelegt ist, ein Eindringen von Luft von außen in das Innenvolumen in Kombination mit einem im Innenvolumen herrschenden Unterdruck zu verhindern, wobei der Unterdruck insbesondere mittels einer an den Luftablass (21) angeschlossenen oder anschließbaren Luftabsaugvorrichtung erzeugbar ist.

3. Abscheider (10) nach Anspruch 1 oder 2, **dadurch gekennzeichnet, dass** eine Verteilervorrichtung vorgesehen ist, mittels welcher Flüssigkeit zum Durchdringen des Dichtelements (50) gegen das mindestens eine Ventilelement (52) drückbar ist, wobei die Verteilervorrichtung vorzugsweise ein rotatorisch antreibbares Element umfasst.

4. Abscheider (10) nach einem der vorhergehenden Ansprüche, **dadurch gekennzeichnet, dass** das Dichtelement (50) zumindest abschnittsweise aus einem elastischen Material, insbesondere Gummi, gefertigt ist, wobei das mindestens eine Ventilelement (52) durch eine Ventilklappe gebildet ist, die durch durchtretende Flüssigkeit insbesondere reversibel nach außen auslenkbar und vorzugsweise einstückig im elastischen Material ausgebildet ist.

5. Abscheider (10) nach Anspruch 4, **dadurch gekennzeichnet, dass** das Dichtelement (50) an einem innerhalb des Innenvolumens angeordneten Stützelement (34) anliegt, in welchem mindestens eine jeweils einer Ventilklappe (52) zugeordnete Durchtrittsöffnung ausgebildet ist, wobei die Durchtrittsöffnung vorzugsweise kleiner ist als die Ventilklappe (52).

6. Abscheider (10) nach einem der vorhergehenden Ansprüche, **dadurch gekennzeichnet, dass** innerhalb des Innenvolumens ein rotatorisch antreibbarer Gemischverteiler (36) drehbar gelagert ist, wobei das aus dem Gemischeinlass (20) tretende Abwassergemisch mittels des Gemischverteilers (36) verteilbar und/oder nach außen schleuderbar ist und wobei der Gemischverteiler (36) vorzugsweise von der Seite gesehen eine im Wesentlichen konische Form aufweist.

7. Abscheider (10) nach Anspruch 6, **dadurch gekennzeichnet, dass** der Gemischverteiler (36) als Laufrad mit einer Mehrzahl von insbesondere geschwungenen Trennschaufeln (37) ausgebildet ist, wobei vorzugsweise das Abwassergemisch über einen ersten Bereich des Laufrads (36) verteilbar und die aus dem Innenvolumen abgesaugte Luft über einen zweiten Bereich des Laufrads (36) zum Luftablass (21) leitbar ist.

8. Abscheider (10) nach Anspruch 6 oder 7, **dadurch gekennzeichnet, dass** der Gemischverteiler (36) mindestens einen durchgängigen Luftkanal (38) umfasst und derart im Bereich der Mündung des Luftablasses (21) angeordnet ist, dass die aus dem Innenvolumen angesaugte Luft durch den mindestens einen Luftkanal (38) in den Luftablass (21) strömt, wobei der mindestens eine Luftkanal (38) vorzugsweise parallel zur Drehachse des Gemischverteilers (36) verläuft.

9. Abscheider (10) nach einem der Ansprüche 6 bis 8, **dadurch gekennzeichnet, dass** der Gemischverteiler (36) zumindest teilweise von einem rotationsstarr mit dem Gehäuse (12) verbundenen Verteilergehäuse (40) umgeben ist, welches mit dem Luftablass (21) und/oder dem Gemischeinlass (20) verbunden ist und mindestens eine Durchtrittsöffnung für das Abwassergemisch aufweist.

10. Abscheider (10) nach einem der vorhergehenden Ansprüche, **dadurch gekennzeichnet, dass** das Gehäuse (12) eine Zentrifugenkammer (16) aufweist, in welcher eine rotatorisch antreibbare Zentrifugentrommel (18) drehbar gelagert ist, wobei der Gemischeinlass (20) und der Luftabzug (21) in die Zentrifugenkammer (16) münden, wobei zwischen der Oberseite der Zentrifugentrommel (18) und dem Gehäuse (12) eine Übertrittsöffnung (24) derart ausgebildet ist, dass während des Zentrifugiervorgangs Flüssigkeit von der Zentrifugentrommel (18) in eine mit dem Flüssigkeitsauslass (22) in Verbindung stehende Auslasskammer (24) gelangen kann und wobei vorzugsweise der Gemischeinlass (20) und/oder der Luftabzug (21) zentral oberhalb der Zentrifugentrommel (18) angeordnet ist.

11. Abscheider (10) nach Anspruch 10 und einem der Ansprüche 6 bis 9, **dadurch gekennzeichnet, dass** der Gemischverteiler (36) innerhalb der Zentrifugentrommel (18) drehbar gelagert und vorzugsweise zusammen mit dieser über eine Welle (42) rotatorisch antreibbar ist, wobei der Gemischverteiler (36) ausgebildet ist, das aus dem Gemischeinlass (20) tretende Abwassergemisch gegen die Innenwandung der Zentrifugentrommel (18) oder gegen das Dichtelement (50) zu schleudern.

12. Abscheider (10) nach Anspruch 10 oder 11, **dadurch gekennzeichnet, dass** an der Zentrifugentrommel (18) im Bereich der Übertrittsöffnung (24) eine Mehrzahl von vorzugsweise auf Umfang verteilten Trennschaufeln (29) angeordnet sind, welche dazu ausgebildet sind, die während des Zentrifugiervorgangs oben aus der Zentrifugentrommel (18) austretende Flüssigkeit nach außen zu schleudern, wobei die Trennschaufeln (29) vorzugsweise an einem Flansch (28) ausgebildet oder angebracht sind, welcher mit einem oberen Rand der Zentrifugentrommel (18) fest oder lösbar verbunden ist.

13. Abscheider (10) nach einem der Ansprüche 10 bis 12, **dadurch gekennzeichnet, dass** das Dichtelement (50) im Bereich der Übertrittsöffnung (24) angeordnet ist und vorzugsweise die Zentrifugentrommel (18) ringförmig umgibt, wobei die Zentrifugenkammer (16) und zumindest ein Abschnitt der Übertrittsöffnung (24) Teil des Innenvolumens sind.

14. Abscheider (10) nach einem der Ansprüche 1 bis 12 und weitergebildet durch die Merkmale des Anspruchs 9, **dadurch gekennzeichnet, dass** das Dichtelement (50) im Bereich der Durchtrittsöffnung des Verteilergehäuses (40) angeordnet ist und vorzugsweise das Verteilergehäuse (40) ringförmig umgibt.

15. Abscheider (10) nach Anspruch 14 und einem der Ansprüche 10 bis 12, **dadurch gekennzeichnet, dass** die Zentrifugentrommel (18) einen Feststoffauslass (23) aufweist, mit welchem ein mit dem Gehäuse (12) abnehmbar verbundener Sedimentationsbehälter (70) zur Sammlung von aus dem Abwassergemisch abgetrennten und nach einem Zentrifugiervorgang unter Schwerkrafteinwirkung absinkenden Feststoffpartikeln verbunden ist, wobei der Abscheider (10) vorzugsweise eine insbesondere gemeinsam mit der Zentrifugentrommel (18) und/oder dem Gemischverteiler (36) antreibbare Pumpe (60) umfasst, mittels welcher eine im Sedimentationsbehälter (70) enthaltene Flüssigkeit zur Abtrennung fester Bestandteile in die Zentrifugentrommel (18) förderbar ist.

## Claims

1. Separator (10) for liquid-air separation of an in particular dental wastewater mixture, comprising a housing (12) having a mixture inlet (20) for the wastewater mixture to be separated, a liquid outlet (22) for liquid separated off from the wastewater mixture, and an air drain (21) for suctioning air out of the interior of the housing (12), wherein a sealing element (50) which is arranged inside the housing (12) and separates the liquid outlet (22) in an airtight manner from an inner volume of the housing (12), which is connected to the air drain (21) and the mixture inlet (20), and **characterised in that** the sealing element (50) comprises at least one valve element (52), which is designed to allow the passage of liquid from the inner volume and to prevent air from entering the inner volume by means of the sealing element (50).

2. Separator (10) according to claim 1, **characterised in that** the at least one valve element (52) is designed to prevent air from the outside from entering the inner volume in combination with a negative pressure prevailing in the inner volume, wherein the negative pressure can be generated in particular by means of an air-suction device which is or can be connected to the air drain (21).

3. Separator (10) according to claim 1 or 2, **characterised in that** a distributor device is provided, by means of which liquid can be pressed against the at least one valve element (52) in order to penetrate the sealing element (50), wherein the distributor device preferably comprises a rotationally drivable element.

4. Separator (10) according to any of the preceding claims, **characterised in that** the sealing element (50) is made of a resilient material, in particular rubber, at least in portions, wherein the at least one valve element (52) is formed by a valve flap which can be deflected outwards, in particular reversibly, by liquid passing through and is preferably integrally formed from the resilient material.

5. Separator (10) according to claim 4, **characterised in that** the sealing element (50) rests against a support element (34) which is arranged inside the inner volume, in which support element at least one passage opening is formed, each of which is associated with a valve flap (52), wherein the passage opening is preferably smaller than the valve flap (52).

6. Separator (10) according to any of the preceding claims, **characterised in that** a rotationally drivable mixture distributor (36) is rotatably mounted inside the inner volume, wherein the wastewater mixture exiting from the mixture inlet (20) can be distributed and/or centrifuged outwards by means of the mixture distributor (36), and wherein the mixture distributor (36) preferably has a substantially conical shape when viewed from the side.

7. Separator (10) according to claim 6, **characterised in that** the mixture distributor (36) is designed as an impeller comprising a plurality of in particular curved separating blades (37), wherein the wastewater mixture can preferably be distributed by a first region of the impeller (36) and the air suctioned in from the inner volume can be conducted to the air drain (21) by a second region of the impeller (36).

8. Separator (10) according to claim 6 or 7, **characterised in that** the mixture distributor (36) comprises at least one continuous air channel (38) and is arranged in the region of the mouth of the air drain (21) such that the air suctioned in from the inner volume flows through the at least one air channel (38) into the air drain (21), wherein the at least one air channel (38) preferably extends in parallel with the axis of rotation of the mixture distributor (36).

9. Separator (10) according to any of claims 6 to 8, **characterised in that** the mixture distributor (36) is surrounded at least in part by a distributor housing (40) which is connected to the housing (12) in a rotationally fixed manner, is connected to the air drain (21) and/or the mixture inlet (20) and comprises at least one passage opening for the wastewater mixture.

10. Separator (10) according to any of the preceding claims, **characterised in that** the housing (12) comprises a centrifuge chamber (16), in which a rotationally drivable centrifuge drum (18) is rotatably mounted, wherein the mixture inlet (20) and the air drain (21) open into the centrifuge chamber (16), wherein a transfer opening (24) is formed between the top of the centrifuge drum (18) and the housing (12) such that, during the centrifuging process, liquid can pass from the centrifuge drum (18) into an outlet chamber (24) connected to the liquid outlet (22), and wherein the mixture inlet (20) and/or the air drain (21) are preferably arranged centrally above the centrifuge drum (18).

11. Separator (10) according to claim 10 and any of claims 6 to 9, **characterised in that** the mixture distributor (36) is rotatably mounted inside the centrifuge drum (18) and can be rotationally drivable, preferably together therewith, via a shaft (42), wherein the mixture distributor (36) is designed to centrifuge the wastewater mixture exiting from the mixture inlet (20) against the inner wall of the centrifuge drum (18) or against the sealing element (50).

12. Separator (10) according to claim 10 or 11, **characterised in that** a plurality of separating blades (29), which are preferably distributed over the circumference and are designed to centrifuge outwards liquid exiting from the top of the centrifuge drum (18) during the centrifuging process, are arranged on the centrifuge drum (18) in the region of the transfer opening (24), wherein the separating blades (29) are preferably formed on or attached to a flange (28) which is rigidly or detachably connected to an upper edge of the centrifuge drum (18).

13. Separator (10) according to any of claims 10 to 12, **characterised in that** the sealing element (50) is arranged in the region of the transfer opening (24) and preferably surrounds the centrifuge drum (18) in an annular manner, wherein the centrifuge chamber (16) and at least one portion of the transfer opening (24) are part of the inner volume.

14. Separator (10) according to any of claims 1 to 12 and developed by the features of claim 9, **characterised in that** the sealing element (50) is arranged in the region of the passage opening of the distributor housing (40) and preferably surrounds the distributor housing (40) in an annular manner.

15. Separator (10) according to claim 14 and any of claims 10 to 12, **characterised in that** the centrifuge drum (18) comprises a solids outlet (23), to which a sedimentation container (70) detachably connected to the housing (12) is connected for collecting solid particles which are separated off from the wastewater mixture and sink under the effect of gravity after a centrifuging process, wherein the separator (10) preferably comprises a pump (60) which is drivable in particular together with the centrifuge drum (18) and/or the mixture distributor (36) and by means of which a liquid contained in the sedimentation container (70) can be conveyed into the centrifuge drum (18) for separating off solid constituents.

## Revendications

1. Séparateur (10) pour la séparation liquide-air d'un mélange d'eaux usées, notamment dentaires, comprenant un boîtier (12) avec une entrée de mélange (20) pour le mélange d'eaux usées à séparer, une sortie de liquide (22) pour le liquide séparé du mélange d'eaux usées et une évacuation d'air (21) pour aspirer l'air de l'intérieur du boîtier (12), un élément d'étanchéité (50) agencé à l'intérieur du boîtier (12) étant prévu, qui sépare de manière étanche à l'air la sortie de liquide (22) d'un volume intérieur du boîtier (12) en liaison avec l'évacuation d'air (21) et l'entrée de mélange (20), et **caractérisé en ce que** l'élément d'étanchéité (50) présente au moins un élément de soupape (52) qui est conçu pour permettre un passage de liquide du volume intérieur vers l'extérieur et pour empêcher une pénétration d'air dans le volume intérieur à travers l'élément d'étanchéité (50).

2. Séparateur (10) selon la revendication 1, **caractérisé en ce que** l'au moins un élément de soupape (52) est conçu pour empêcher une pénétration d'air de l'extérieur dans le volume intérieur en combinaison avec une dépression régnant dans le volume intérieur, la dépression pouvant être générée notamment au moyen d'un dispositif d'aspiration d'air raccordé ou pouvant être raccordé à l'évacuation d'air (21).

3. Séparateur (10) selon la revendication 1 ou 2, **caractérisé en ce qu'**il est prévu un dispositif de distribution au moyen duquel du liquide peut être pressé contre l'au moins un élément de soupape (52) pour traverser l'élément d'étanchéité (50), le dispositif de distribution comprenant de préférence un élément pouvant être entraîné en rotation.

4. Séparateur (10) selon l'une quelconque des revendications précédentes, **caractérisé en ce que** l'élément d'étanchéité (50) est fabriqué au moins par sections en un matériau élastique, notamment en caoutchouc, l'au moins un élément de soupape (52) étant formé par un clapet de soupape qui peut être dévié vers l'extérieur, notamment de manière réversible, par le liquide qui le traverse et qui est de préférence réalisé d'une seule pièce dans le matériau élastique.

5. Séparateur (10) selon la revendication 4, **caractérisé en ce que** l'élément d'étanchéité (50) s'appuie sur un élément de support (34) agencé à l'intérieur du volume intérieur, dans lequel est réalisé au moins une ouverture de passage associée respectivement à un clapet de soupape (52), l'ouverture de passage étant de préférence plus petite que le clapet de soupape (52).

6. Séparateur (10) selon l'une quelconque des revendications précédentes, **caractérisé en ce qu'**un distributeur de mélange (36) pouvant être entraîné en rotation est monté à rotation à l'intérieur du volume intérieur, le mélange d'eaux usées sortant de l'entrée de mélange (20) pouvant être distribué et/ou projeté vers l'extérieur au moyen du distributeur de mélange (36), et le distributeur de mélange (36) présentant de préférence une forme essentiellement conique lorsqu'il est vu de côté.

7. Séparateur (10) selon la revendication 6, **caractérisé en ce que** le distributeur de mélange (36) est réalisé sous forme de roue mobile avec une pluralité d'aubes de séparation (37), notamment incurvées, le mélange d'eaux usées pouvant de préférence être distribué par l'intermédiaire d'une première zone de la roue mobile (36) et l'air aspiré du volume intérieur pouvant être dirigé vers l'évacuation d'air (21) par l'intermédiaire d'une deuxième zone de la roue mobile (36).

8. Séparateur (10) selon la revendication 6 ou 7, **caractérisé en ce que** le distributeur de mélange (36) comprend au moins un canal d'air continu (38) et est agencé dans la zone de l'embouchure de l'évacuation d'air (21) de telle sorte que l'air aspiré à partir du volume intérieur s'écoule à travers l'au moins un canal d'air (38) dans l'évacuation d'air (21), l'au moins un canal d'air (38) s'étendant de préférence parallèlement à l'axe de rotation du distributeur de mélange (36).

9. Séparateur (10) selon l'une quelconque des revendications 6 à 8, **caractérisé en ce que** le distributeur de mélange (36) est au moins partiellement entouré par un boîtier de distribution (40) relié de manière solidaire en rotation au boîtier (12), qui est relié à l'évacuation d'air (21) et/ou à l'entrée de mélange (20) et qui présente au moins une ouverture de passage pour le mélange d'eaux usées.

10. Séparateur (10) selon l'une quelconque des revendications précédentes, **caractérisé en ce que** le boîtier (12) présente une chambre de centrifugation (16) dans laquelle est monté à rotation un tambour de centrifugation (18) pouvant être entraîné en rotation, l'entrée de mélange (20) et l'extraction d'air (21) débouchant dans la chambre de centrifugation (16), une ouverture de transfert (24) étant réalisée entre le côté supérieur du tambour de centrifugation (18) et le boîtier (12) de telle sorte que, pendant le processus de centrifugation, du liquide peut passer du tambour de centrifugation (18) dans une chambre de sortie (24) en liaison avec la sortie de liquide (22), et de préférence l'entrée de mélange (20) et/ou l'extraction d'air (21) est agencée de manière centrale au-dessus du tambour de centrifugation (18).

11. Séparateur (10) selon la revendication 10 et l'une quelconque des revendications 6 à 9, **caractérisé en ce que** le distributeur de mélange (36) est monté à rotation à l'intérieur du tambour de centrifugation (18) et peut être entraîné en rotation, de préférence conjointement avec celui-ci, par l'intermédiaire d'un arbre (42), le distributeur de mélange (36) étant réalisé pour projeter le mélange d'eaux usées sortant de l'entrée de mélange (20) contre la paroi intérieure du tambour de centrifugation (18) ou contre l'élément d'étanchéité (50).

12. Séparateur (10) selon la revendication 10 ou 11, **caractérisé en ce que** sur le tambour de centrifugation (18), dans la zone de l'ouverture de transfert (24), sont agencées une pluralité d'aubes de séparation (29), de préférence réparties sur la circonférence, qui sont réalisées pour projeter vers l'extérieur le liquide qui sort par le haut du tambour de centrifugation (18) pendant le processus de centrifugation, les aubes de séparation (29) étant de préférence réalisées ou installées sur une bride (28) qui est reliée de manière fixe ou amovible à un bord supérieur du tambour de centrifugation (18).

13. Séparateur (10) selon l'une quelconque des revendications 10 à 12, **caractérisé en ce que** l'élément d'étanchéité (50) est agencé dans la zone de l'ouverture de transfert (24) et entoure de préférence le tambour de centrifugation (18) de manière annulaire, la chambre de centrifugation (16) et au moins une section de l'ouverture de transfert (24) faisant partie du volume intérieur.

14. Séparateur (10) selon l'une quelconque des revendications 1 à 12 et perfectionné par les caractéristiques de la revendication 9, **caractérisé en ce que** l'élément d'étanchéité (50) est agencé dans la zone de l'ouverture de passage du boîtier de distribution (40) et entoure de préférence le boîtier de distribution (40) de manière annulaire.

15. Séparateur (10) selon la revendication 14 et l'une quelconque des revendications 10 à 12, **caractérisé en ce que** le tambour de centrifugation (18) présente une sortie de matières solides (23) à laquelle est relié un récipient de sédimentation (70) relié de manière détachable au boîtier (12) pour la collecte de particules solides séparées du mélange d'eaux usées et tombant sous l'effet de la gravité après un processus de centrifugation, le séparateur (10) comprenant de préférence une pompe (60) pouvant être entraînée notamment conjointement avec le tambour de centrifugation (18) et/ou le distributeur de mélange (36), au moyen de laquelle un liquide contenu dans le récipient de sédimentation (70) peut être transporté dans le tambour de centrifugation (18) pour la séparation des constituants solides.
